# EUROPEAN PATENT SPECIFICATION

(11) **EP 4 445 948 B1**
(45) Date of publication and mention of the grant of the patent: **04.11.2026**
(21) Application number: 24168216.0
(22) Date of filing: 03.04.2024
(51) Int. Cl.: A61N 2/02

(54) **MEDICAL SYSTEM FOR THERAPEUTIC APPLICATIONS BY MEANS OF USE OF MAGNETIC INDUCTION**
MEDIZINISCHES SYSTEM FÜR THERAPEUTISCHE ANWENDUNGEN MITTELS MAGNETISCHER INDUKTION
SYSTÈME MÉDICAL POUR APPLICATIONS THÉRAPEUTIQUES PAR L'UTILISATION D'INDUCTION MAGNÉTIQUE

(30) Priority: 13.04.2023 IT 202300007074
(43) Date of publication of application: 16.10.2024
(73) Proprietor: Ami Medtech S.r.l., 00163 Roma (RM) (IT)
(72) Inventor: MANTARRO, Marco, I-00163 ROMA (RM) (IT)
(74) Representative: Fiammenghi, Eva

(56) References cited:
- WO-A1-01/15775
- US-A1- 2010 280 303
- US-A1- 2014 081 073
- US-A1- 2017 095 199
- US-A1- 2022 072 327
- US-A1- 2023 104 434

## Description

### Field of application

The invention refers to the field of medical devices. More in detail the present invention describes a system for use in the administration of therapies with electromagnetic fields applied to extended or localized zones.

### Prior art

Therapy with magnetic fields is conventionally used for orthopedic pathologies; in the last few years, several studies have shown the usefulness also in other pathologies such as, for example, those neurological. The magnetic fields generally employed in these therapies are fields of different kind and nature, from low frequencies ELF (Extreme Low Frequency), to high frequencies and high intensities; i.e. fields with extremely low frequencies comprised between 0.1 Hz and 250 Hz and high-frequency fields on the order of MHz, and high intensities on the order of several hundred Gauss. The magnetic signals of a therapeutic apparatus that produces ELF fields trigger a series of chemical and physical reactions that are capable of accelerating the enzymatic kinetics in that series of functions, known overall as Repair Morphogenesis (MR).

Over the years, many patents have proposed apparatuses, systems and methods for solving problems of various nature connected to the administration of a therapy with electromagnetic fields.

One example is the object of the patent application JP2022021025A by H. TOSHIKATSU, I. TATSUYA, N. KEITA. The invention describes a medical device with magnetic induction. Specifically the invention describes a device with which it is possible to administer an electromagnetic induction therapy to a patient, who inserts the part to be administered the therapy in a cylindrical cable. The invention also solves the problem of overheating through a system of self-turning off and temperature detection.

Medical apparatuses like that described by the invention abovementioned invention have large size and are of course not very practical in the case of patients that are not self-sufficient. In addition, this apparatus type has the limitation of allowing the treatment only in localized zones, and often only in one zone at a time. A therapeutic treatment, however, often requires the application on multiple parts of the body simultaneously. Moreover, it is obvious that large-size apparatuses like those mentioned above are poorly efficient with regard to the treatment of multiple patients of a clinic and/or division.

One solution to these problems has been given by wearable devices for the treatment with electromagnetic induction. The patents have in fact claimed, over the years, devices wearable as bands (WO2006060304A2), helmets (US2020376288A1) and still others. Publications US 2023/104434 A1 and US 2022/072327 A1 describe treatment systems for electromagnetic therapy being controllable through user interfaces on mobile devices.

The inventions reported up to now as a mere non-exhaustive example are representative of the inventive-technological situation at present.

The inventions reported all have devices which due to their particular shape, structure type and/or management mode cannot be employed in a versatile manner for producing magnetic fields, adapted and/or inserted in various supports such as smartphones, tablets, accessories but also directly in the human body, nor can they carry a magnetic field induced through different mediums such as water. None of the abovementioned inventions proposes solutions to the technical problem, in the medical field, of programming and managing, remotely, with a single control unit, the magnetotherapy treatments to be carried out on multiple patients, also with limited motor capacities. None of the abovementioned inventions provide systems capable of automatically interrupting a therapeutic treatment in the case of detected physiological conditions of discomfort/illness in a user.

### Summary of the invention

The problems set by the prior art were resolved with the system according to the present invention defined by the claims.

Object of the present invention is to provide a system for use in the administration of medical therapies on different parts of the body by means of the magnetic induction with the use of non-ionized radiations through a miniaturized system, also wearable throughout the body. According to the present invention, a system is attained which exploits the magnetic induction in order to transfer, to biological tissues, bioactive information packets with applicability in various fields.

With "bioactive information packets" it is intended magnetic signals induced by the system and by the devices that compose it which are capable of stimulating a biological response in the subject to whom they are administered, and they are defined "packets" since they contain information on four fundamental parameters for the magnetic signals which are intensity, frequency, duration and waveform.

Preferably the system, according to the invention, is attained for use in the administration of therapies with magnetic induction for users affected by specific pathologies, both in the field of pain therapy and in Regenerative Medicine.

More in detail, the apparatus according to the present invention was especially designed for carrying out magneto-therapeutic ELF (Extreme Low Frequency) applications at low intensity (comprised between 1 and 220 µT) in professional setting and otherwise.

Specifically the system of the invention comprises a hardware part and optionally a software part.

The hardware part comprises a dispenser of electromagnetic field and a control unit which allows selecting the type of treatment/program and sending an input to the dispenser in order to start the treatment itself.

More in detail, the dispenser comprises at least: a power supply circuit which accepts, in input, the rechargeable batteries contained within; a receiving and generating unit which acquires the data relative to the treatment/program through a connection (by way of non-limiting example, Bluetooth, Wi-Fi and/or other) and uses said data for generating the electromagnetic field.

In one embodiment, the system according to the invention comprises at least a dispenser of magnetic fields, in turn comprising a power supply circuit for rechargeable batteries, a receiving and generating unit which receives one or more signals and transmits a current into windings of conductive material contained within in order to generate one or more ELF magnetic fields with frequency comprised between 0.1 and 250 Hz, and intensity comprised between 1 µT and 220 µT; all the various units are contained in said dispenser preferably having the shape of a parallelepiped and width comprised between 20mm and 110mm, length comprised between 30mm and 150mm and thickness comprised between 10mm and 40mm. The invention further comprises at least a control unit comprising an interface for managing the treatments, a communication unit which sends inputs to said receiving and generating unit of the dispenser in order to start one or more treatments.

The system further comprises a connection unit and a web portal and/or application; said connection unit connecting to said web portal and/or application; said web portal and/or application for purchase and/or acquisition and management of treatments comprises:
- a marketplace section in which, by means of selection, said treatments are purchased and/or obtained;
- a virtual blister section in which the treatments are preserved and from which one or more treatments are selected for their administration;
- a treatment section in which the treatment is monitored during administration;
- a management section in which the start and interruption of a treatment are programmed at specific times.

The system according to the present invention is intended for use in the administration of therapies through magnetic induction on one or more users, by means of one or more treatments.

In another form of use of the present invention, the dispenser is employed for exposing water to ultra-weak sources of electromagnetic fields. The advantages offered by the present invention are evident in light of the description set forth up to now and will be even clearer due to the enclosed figures and to the relative detailed description.

### Description of the figures

The invention will be described hereinbelow in at least a preferred embodiment as a non-limiting or non-binding example with the aid of the enclosed figures, in which:
- FIGURE 1 shows a general view of the system 100 for magnetic induction;
- FIGURE 2 shows a view of the system 100 comprising two wearable modular suits 300, each with a dispenser 110 that is interfaced with the same control unit 120;
- FIGURE 3 shows several screens of a web portal and/or application 200 comprised in the system 100;
- FIGURE 4 shows a general view of the system 100 comprising a single device 130.

### Detailed description of the invention

The main function of the therapeutic devices is that of emitting, in proximity to the body of a patient, a complex electromagnetic field of ELF type whose characteristics are varied, through algorithms which determine different patterns of electromagnet waves to be emitted in order to obtain different therapeutic goals. The electromagnetic signals ELF dispensed by the present invention in fact stimulate a series of chemical and physical signals and are capable of accelerating the enzymatic kinetics of the Repair Morphogenesis (MR) functions.

According to the present invention, a system 100 for magnetic induction is provided. The invention is preferably, but not exclusively, employed in the medical-therapeutic field, this is in fact effective for use in the therapeutic treatment of different pathologies, as a non-limiting example pathologies associated with pain such as arthrosis, rheumatoid arthritis, spondyloenthesoarthritis, ankylosing spondylitis, extra-articular rheumatisms, gout, in the treatment of edema, phlogosis, bacterial infections and in regenerative medicine in general. The system according to the present invention is preferably but not exclusively intended for a therapeutic, anti-pain and regenerative use in the orthopedic field, physiatrics, traumatology, sports medicine and in regenerative medicine in general. Other fields of application are, by way of a non-limiting example, those of determatology, aesthetic medicine, dentistry, ophthalmology and podology. Another field of application is the veterinarian field, since therapies with magnetic induction are not dangerous for animals.

The system 100 is used for the administration of therapies with magnetic induction on one or more users 1 by means of one or more treatments 10.

By treatment/program 10, it is intended the group of bioactive information packets or of packets containing the information on four parameters which are intensity, frequency, waveform and duration of the magnetic field that is produced with a dispenser 110; such information can for example be contained in digital algorithms. Each treatment/program 10 contains two or more bioactive information packets. Each treatment 10 then provides for the delivery of an electromagnetic field ELF (Extremely Low Frequency) with frequency comprised between 0.1 and 250 Hz, and intensity comprised between 1 µT and 220 µT in proximity to and/or directly in contact with the user 1.

The system according to the invention consists of a hardware part and of a software part.

The hardware part comprises an electromagnetic field dispenser and a control unit which allows selecting the type of treatment and sending an input to the dispenser in order to start the treatment itself.

According to the invention and as will be better detailed hereinbelow, the dispenser and the control unit can be components of a same device or they can be parts of separate devices.

By dispenser 110 it is intended a hardware device that dispenses magnetic fields and comprises a power supply circuit 111 for rechargeable batteries, a receiving and generating unit 112 which receives one or more signals and transmits a current to windings of conductive material 113. More in detail the dispenser said power supply circuit 111 accepts, at the input, rechargeable batteries contained therein; said receiving and generating unit 112 which acquires the data relative to the treatment 10 through a connection (by way of non-limiting example, Bluetooth, Wi-Fi and/or other) and uses said data for generating an electromagnetic field.

The dispenser 110 according to the present invention is attained in reduced dimensions (width comprised between 20mm and 110mm, length between 30mm and 150mm and thickness between 10 and 40mm) which therefore allow the insertion thereof into objects of daily use (as a non-limiting or non-binding example, smartphones, tablets) in wearable clothing and/or accessories (as a non-limiting or non-binding example, bracelets, necklaces, watches).

Said dispenser is inscribed in an elliptical ring (i.e. a ring with semicircular section that is extended along an ellipse) in order to increase the area on which a magnetotherapy treatment is carried out. Said parallelepiped is inscribed within the empty elliptical space described by the ring which has (internal) dimensions which provide for a major axis comprised between 40mm and 160mm and a minor axis comprised between 30mm and 120mm. The parallelepiped inscribed in the elliptical ring contains said power supply circuit 111 and said receiving and generating unit 112, while said windings of conductive material 113 are inserted within the thickness of the body of the elliptical ring with semicircular section that describes the ellipse whose dimensions have been defined above.

The dispenser can be attained in various forms, preferably that of a parallelepiped, and in any case has dimensions comprised in the following intervals:
- width between 2.0 and 11.0 cm;
- length between 3.0 and 15.0 cm;
- thickness from 1.0 to 4.0 cm.

In one embodiment of the present invention, the reduced dimensions allow inserting the dispenser within electronic devices such as cell phones, tablets, smart watches, other electronic devices or wearable devices in general.

The dispenser is formed by one or more circuit boards (by way of a non-binding example with SMD components) that transfer the signal to one or more windings of enameled copper wire and/or other conductive material.

In various forms of application of the present invention, the windings of conductive material can also be outside the dispenser and used in accessories, clothing and other wearable devices. In one embodiment of the present invention, the reduced dimensions of the dispenser allow creating wearable accessories which integrate dispensers and copper windings.

In the embodiments in which the windings of conductive material are outside the dispenser, this has a central body that comprises only the power supply circuit for the rechargeable batteries and the unit for receiving the signal and generating the magnetic field that will be generated by means of circulation of current in the external windings of conductive material. In one embodiment of the present invention, the dispenser is inserted in clothing that comprise, at their interior, the windings of conductive material such to allow the generation and the propagation of the magnetic field ELF.

In one embodiment of the present invention, the dispenser is connected or comprised in a wearable modular suit within which one or more circuit boards are present which transfer the signal to one or more windings of enameled copper wire and/or other conductive material. The windings of conductive material wire, within which a current lower than 0.1A is made to flow by means of the application of a voltage which is less than 5V, are isolated and then enclosed in further isolating protections: this prevents, in a safe and complete manner, the contact with the human body, also in the case of serious damage of the protections. The circuits are easily interpretable by any expert technician of the field, and therefore their characteristics are omitted from the present description, taking for granted that the man skilled in the art understands their presence and their function. The electromagnetic field generated by the dispenser and by the windings of conductive material varies based on the indications in the programs/treatments dedicated to each pathology. All the parameters (intensity of the field, frequency, waveforms, duration of the treatment and others) relative to the treatments are predetermined and are transmitted to the electromagnetic dispenser by a web portal and/or application. The wearable suit is defined modular since it comprises multiple parts (by way of non-binding example: gloves, sleeves, shirt, pants, socks, hat and/or others) each comprising one or more power connectors that allow the direct attachment of the dispenser to the selected part of the suit, and one or more joining connectors that allow joining multiple parts of the suit together.

In one embodiment of the present invention, the parts of the wearable modular suit can be connected to each other through a connection cable that joins the respective connectors by joining. In this embodiment, it is possible to simultaneously carry out treatments on distant parts of the body such as, for example, a hand and a food without having to necessarily put on the entire suit, but by wearing only a glove and a sock and connecting them through the connection cable. The pulse thus provided by the dispenser will pass from one part to the other through the connection cable.

In one embodiment, the dispenser, by connecting multiple parts of the wearable suit, through the connection cables, comprises multiple inputs so as to allow the coupling of multiple connection cables and the sending of independent electromagnetic pulses to the windings of conductive material of each part of the wearable suit so as to be able to carry out different and independent treatments on multiple parts of the body simultaneously.

The control unit according to the present invention can for example be a computer, a smartphone or a tablet with which, remotely, a user (such as for a doctor, a health worker and/or others) can provide the input to the dispenser in order to start a treatment.

In one embodiment of the present invention, the control unit can also control more than one dispenser so as to allow remotely, through a single interface, the activation, the interruption and the management of the treatments to be carried out on multiple patients.

The control unit allows the users to be interfaced with the software part of the invention. The software part consists of an application and/or a web portal. The web portal and/or the application has the primary function of allowing users to start, pause, interrupt and monitor a treatment by communicating with the dispenser.

The communication occurs, as already seen, between the control unit and the dispenser through Bluetooth, Wi-Fi (and/or other wireless communication protocols) in the embodiments in which the control unit is separated from the dispenser. In the embodiments of the invention in which the control unit and the dispenser are physically connected and/or are components of a single hardware device, the control unit comprises a touchscreen (or other similar technology) in order to directly access the web portal and/or application.

The web portal and/or application allows, in addition to the management of one or more treatments, in one embodiment, purchasing and/or reselling the treatments, thus giving the possibility to users to be able to exchange possible non-used treatments, eliminating waste and recovering part of the price sustained for the purchase. In a suitable section, the users can purchase the treatments that are thus preserved in a virtual blister section. From the virtual blister section, the user can select the treatment to be administered, which is thus used up.

The web portal and/or application allows, in the embodiments in which the control unit communicates with multiple dispensers (as a non-limiting or non-binding example, in a hospital clinic), to start multiple treatments, also simultaneously, on different users who wear, as a non-limiting example, different wearable modular suits or applicators of another kind.

The web portal and/or application also allows programming the automatic starting of treatments in specific times, or the reception of particular notifications. As a non-limiting or non-binding example, the web portal and/or application can be programmed in order to start a treatment when the status of the user who wears the wearable modular suit changes from "engaged in control visit" to "rest - in room".

The treatments can be sold, purchased and exchanged by means of web portal and/or application, as they are information, for example, in algorithms regarding intensity, frequency, waveform, duration (and possible other data) of the electromagnetic field to be produced in order to produce a therapeutic effect.

In one embodiment of the invention, said treatments 10 and/or the bioactive information packets are created with blockchain technology and/or as NFT to which a smart contract can be connected in order to unlock other services and/or functions. The unlock of accessory functions that can be, as a non-limiting or non-binding example, bound to ensure a percentage of payment to the treatment 10 sellers, occurs through implemented validation algorithms of the web portal and/or application, capable of verifying the veracity of a smart contract and consequent executing actions with a cause-effect computer logic.

In light of that described above, it is understood that the invention is also adapted for use in the aerospace field. The wearable modular suit can in fact be worn by astronauts, also under a spatial suit during extravehicular activities, such to be able to autonomously administer or be able to administer their treatments when/if necessary. In the case of permanence in space for long periods, the invention has proven particularly useful since it allows an unlimited use of treatments, overcomes the problem of preservation of drugs tied to their expiry, and also allows obtaining new and more effective treatments via radio, during the communications with the stations on the ground.

In another embodiment of the present invention, the dispenser can be directly inserted within space suits by connecting the control unit to the unit for controlling suit operations.

In a form of use of the present invention, the reduced dimensions of the dispenser, the fact that this is made with an airtight coating of biocompatible materials (i.e. of materials such to not be harmful for a living organism) as a non-limiting or non-binding example polycarbonates and polymers and its electrical properties (such as for example the intensity of current at its interior) allow being able to implant it within living organisms in order to administer the treatments remotely via Bluetooth, Wi-Fi and/or other communication protocols, also to subjects who are not self-sufficient or cannot autonomously execute the administration of the treatment, preventing the wearing of wearable devices. The dispenser is defined implantable, therefore, since it possesses mechanical, physical, chemical and electrical properties such to not damage the biological systems and to not be in turn damaged after its insertion within a living organism or it contact therewith or with its fluids.

In other embodiments of the present invention, the dispenser is employed in order to administer said treatments on animals. In these embodiments, in addition to the possible implementations described above, the dispenser and/or the dispenser together with the control unit are inserted in collars, vests, saddles and/or other clothing that the animals can wear or devices that can be used in proximity to an animal.

In another form of use of the present invention, the dispenser is employed in order to expose water, preferably drinkable water, to ultra-weak sources of electromagnetic fields. The water molecules, even if they have the same chemical characteristic of being composed of two atoms of hydrogen and one atom of oxygen (H₂O), appear in two different forms. In the first case, they are independent from each other and freely floating, in the other case they are organized into structures characterized by a collective rhythm of vibrational oscillation which is indicated with the name Coherence Domain. The Coherence Domains come into direct resonance with electromagnetic fields of specific frequencies. The exposure of the water to specific electromagnetic fields (like those generated with the present invention) allows "informing it" i.e. ensuring that this can carry a particular type of information. In this manner, by means of exposure to one or more magnetic fields, it is possible to make the water pick up specific therapeutic properties that can be transferred to the organism by taking it orally.

The present invention will now be illustrated as a non-limiting or non-binding example, with reference to the figures which illustrate several embodiments relative to the present inventive concept.

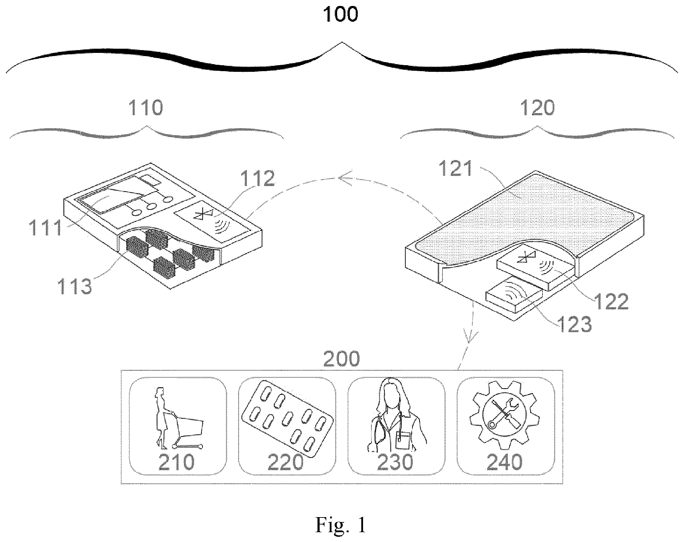

With reference to FIG. 1, a general view of said system 100 is shown for use in the administration of therapies with magnetic induction according to the present invention. In FIG. 1, as in the following description, the embodiment of the present invention is illustrated which is deemed today to be the best.

Said system 100 comprises:
- at least a magnetic field dispenser 110, in turn comprising a power supply circuit 111 for rechargeable batteries, a receiving and generating unit 112 which receives one or more signals and transmits a current into windings 113 of conductive material in order to generate the magnetic field ELF; the windings 113 made of enameled copper and/or another conductive material are isolated and then enclosed in further isolating protections: this safely and completely prevents contact with the human body, also in the case of serious damage of the protections; within the latter, a current lower than 0.1A is made to flow, by means of the application of a voltage which is less than 5V;
- at least a control unit 120 comprising an interface 121 for managing the treatments 10, a communication unit 122 which sends inputs to said receiving and generating unit 112 of the dispenser 110 in order to start the treatments 10, a connection unit 123 in order to be connected to
- a web portal and/or application 200;
- at least a web portal and/or application 200 for the purchase and/or acquisition, and the management of treatments 10;
said web portal and/or application 200 comprising:
- a marketplace section 210 in which, by means of prescription, said treatments 10 are purchased and/or obtained;
- a virtual blister section 220 in which the treatments 10 are preserved and from which one or more treatments 10 are selected for their administration;
- a treatment section 230 in which the treatment 10 are monitored during administration;
- a management section 240 in which the start and interruption of a treatment 10 are programmed at specific times.

The treatments 10 are therefore purchased, acquired and/or sold by means of the web portal and/or application 200. This characteristic of our invention allows solving the technical problems tied to the perishability of the drugs. Having "digital drugs" or medications in algorithm form in fact allows being able to make use of them at any moment, even years after their purchase, since they cannot deteriorate.

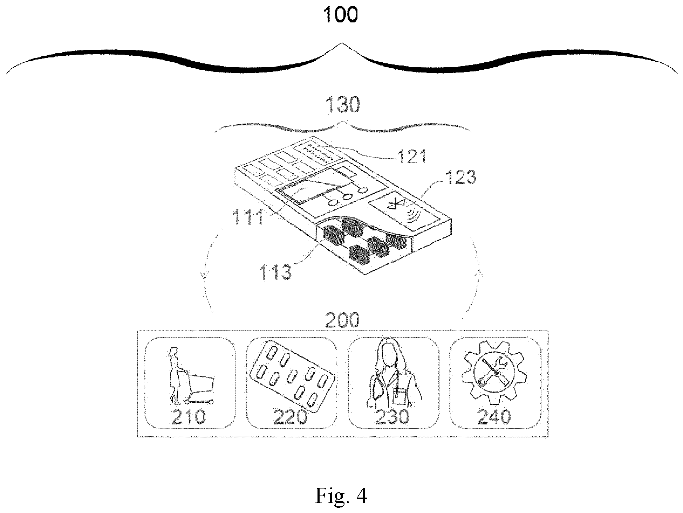

In other embodiments of the present invention, the dispenser 110 and the control unit 120 are included in a single device 130 (shown in FIG. 4). By single device 130 it is intended a hardware in which said dispenser 110 and said control unit 120 are joined and are not separable except by tampering and/or disassembly. Said single device 130 is an independent device, this in fact allows the navigation in said web portal and/or application 200 as well as the administration of the treatments 10. This does not depend on other devices, comprising at its interior both said dispenser 110 and said control unit 120 which, as already described, comprises an interface 121 and a connection unit 123.

In several embodiments of the present invention, the connection unit 123 is a housing for a SIM card with relative SIM card capable of ensuring the connection and navigation in said web portal and/or application 200.

In one embodiment of the present invention, the dispenser 110 is connected to a wearable modular suit 300 within which one or more circuit boards are present which transfer the signal to one or more windings 113 of enameled copper wire and/or other conductive material. The wearable modular suit 300 is defined modular since it comprises multiple parts 310 (by way of non-binding example: gloves, sleeves, shirt, pants, socks, hat and/or other items) that can be used simultaneously, separately or in various combinations. Each part 310 of the wearable suit in fact comprises one or more power connectors 330 which allow the direct attachment of the dispenser 110 or the single device 130 to the selected part 310 of the suit 300, and one or more joining connectors 320 which allow joining multiple parts 310 of the suit 300 to each other. In this manner, it is possible to wear the suit 300 in all its parts 310 in order to have a treatment linked to the entire body. In light of that just described, there are obvious possible combinations obtainable by wearing various parts 310 of wearable modular suit 300, and it should be underlined that the control unit 120 not only enables selecting specific parts 310 of the body on which intervention can occur, but it also allows carrying out different treatments 10 on different parts 310 of the wearable modular suit 300.

In one embodiment, the wearable modular suit 300 comprises user sensors 350 (not shown in the figures) which, being proximal to and often in contact with the body of the user 10, are able to measure physiological and biochemical parameters thereof such as, as a non-limiting or non-binding example, heart rate, blood pressure, temperature, sweating and/or other parameters. These user sensors 350 are connected to the dispenser 110 which, in this embodiment, comprises a transmission unit 115 (not shown in the figures) for the data, which has the task of transmitting the measurements of the parameters of the user 1 to said control unit 120 and/or to said web portal and/or application 200 for managing the treatments 10.

The web portal and/or application 200, in said treatment section 230 also allows monitoring the measurements of the physiological and biochemical parameters of the user 1 carried out with the user sensors 350. In this embodiment, the web portal and/or application 200 can be programmed so as to interrupt a treatment in the event in which the physiological and biochemical parameters of the user 1 are outside established ranges and personalizable (as a non-limiting or non-binding example the temperature range can be fixed between values 35 - 37°C), and to send notification on the control unit 120.

The present invention then provides a solution to an even more significant problem, that of quickly and simultaneously managing multiple users 1 on which multiple treatments 10 are to be carried out. This effect is reached when multiple dispensers 110 are connected to a single control unit 120 which communicates with the web portal and/or application 200.

In various embodiments of the present invention, the control unit 120 can be a computer, a smartphone or a tablet with which, remotely, an operator 2 (such as a doctor, a health worker and/or others) can provide the input to the dispenser 110 in order to start a treatment 10.

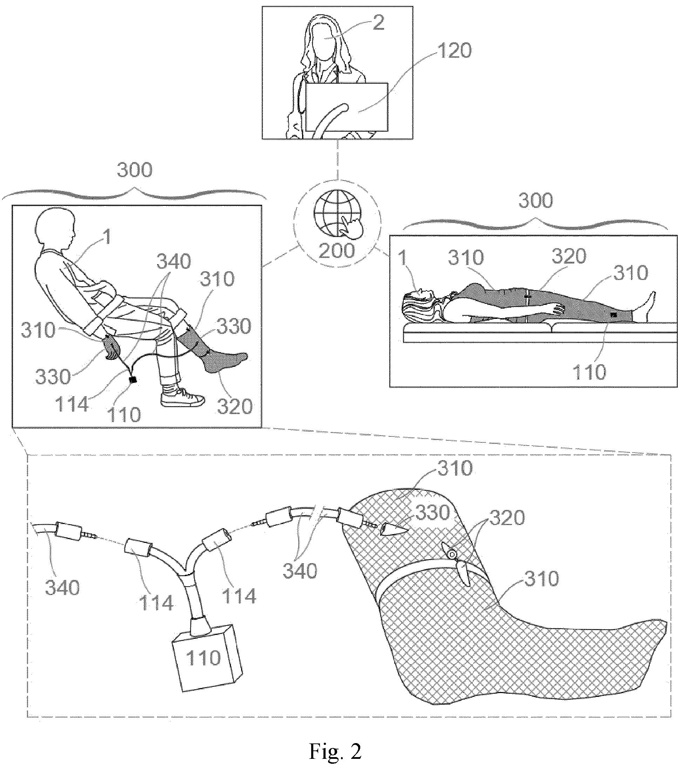

With reference to FIG. 2, a view of the system 100 is shown comprising wearable modular suits 300, each with a dispenser 110 that is interfaced with the same control unit 120.

FIG. 2 will be described hereinbelow with reference to FIGS. 1 and 2.

Said wearable modular suits 300 each comprise multiple parts 310 that can be coupled together by means of one or more joining connectors 320. The joining connectors 320 are coupling devices in which, surrounded by isolating material, there is the joining between two more electric circuits connected to the windings 113 (not shown in FIG. 2) of conductive material (the joining connector 320 is for example a connector of male-female Jack type, as can be observed in the enlargement at the bottom of FIG. 2). In this manner, by wearing multiple parts 310 connected together with the joining connectors 320, only one dispenser 110 is necessary in order to send the electrical pulse that generates the magnetic field ELF, to all the parts 310.

Each part 310 is connected to the dispenser 110 through one or more power connectors 330, and said parts 310 connected to said dispenser 110 with one or more power connectors 330. Said power connectors 330 allow the connection of the dispenser 110 with the part 310. The power connectors 330 place the receiving and generating unit 112 (shown in FIG. 1) of the dispenser 110 in contact with the windings 113 (not shown in FIG. 2) present in the part 310 to which the dispenser 110 is directly connected. When other parts 310 are connected by means of said joining connectors 320 to the part 310 to which the dispenser 110 has been directly connected, the current dispensed from the receiving and generating unit 112 of the dispenser 110 also reaches these other parts 310, generating a magnetic field through the windings 113 present in each of these. Said power connector 330 is for example of male-female Jack type. In FIG. 2 in particular it is possible to observe that an operator 2 from a single control unit 120 (in FIG. 2 represented by a computer) manages the treatments 10 over two different users 1 simultaneously. Specifically the user 1 in FIG. 2, on the left, wears three parts 310 of wearable modular suit 300 which are a glove, a part that covers a shin and a part that covers a foot. The part 310 that covers the left foot and the part that covers the left shin are connected to each other through said joining connector 320. The part that covers the left shin is connected to the dispenser 110 through the connection power connector 330 - connection cable 340 - input 114. The dispenser 110 shown in FIG. 2 on the left (and in the enlargement on the bottom of FIG. 2) has two inputs 114 connected to two connection cables 340 in order to connect separate parts 310 of the wearable modular suit 300. In the configuration shown in FIG. 2 on the left, the user 1 can be subjected to two simultaneous and different therapies for the right hand and for the lower part of the left leg. On the bottom of FIG. 2, it is possible to observe an exploded enlargement of the dispenser 110, of the inputs 114, of the connection cables 340, of the power connectors 330, of the parts 310 and of the joining connectors 320.

In FIG. 2 on the right, one observes a user 1 that wears the parts 310 of the wearable modular suit 300 covering chest and legs. In FIG. 2 on the right, the dispenser 110 is connected directly to a part 310 of the wearable modular suit 300 that covers the right leg. The two parts 310 shown in FIG. 2 on the right are connected to each other by means of said joining connector 320.

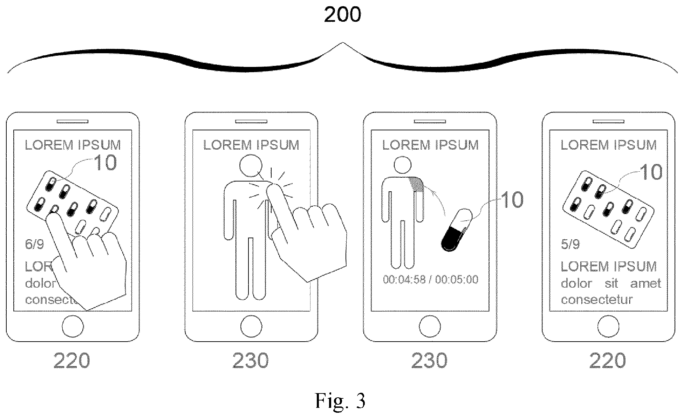

With reference to FIG. 3, several screens of said web portal and/or application 200 are shown. FIG. 3 will be described hereinbelow with reference to FIGS. 1, 2 and 3.

The first screen on the left in FIG. 3 shows the virtual blister section 220 in which there is the indication of the treatments 10 remaining (reported as an example in FIG. 3: "6/9"). By selecting a treatment from the first screen on the left, the web portal and/or application 200 proceeds in the treatment section 230 (second screen from the left in FIG. 3) in which it shows scheme of the person, giving the possibility of selecting (through the touch as shown in FIG. 3) the part of the body in which the treatment is carried out. In the third screen from the left in FIG. 3, the treatment section 230 shows a countdown of the remaining time for completing the treatment. In the first screen on the right in FIG. 3, it is shown that, by returning on the virtual blister section 220 of the web portal and/or application 200, a lower number of remaining available treatments 10 is shown (in the case of FIG. 3, first screen from the right, "5/6").

With reference to FIG. 4, a general view is shown of said system 100 comprising said single device 130 according to the present invention.

It is observed that the single device 130 shown in Figure 4 is provided with an interface 121 comprising displays and various buttons for allowing the control thereof as well as the turning on and off thereof. The single device 130 shown in Figure 4 comprises said power supply circuit 111 for rechargeable batteries, said connection unit 123 which is connected with said web portal and/or application 200 and receives the treatments 10 thereof in order to then transmit a current to said windings of conductive material 113.

Finally, it is clear that modifications, additions or variations that are obvious for the man skilled in the art can be made to the invention, without departing from the protective scope that is provided by the enclosed claims.

## Claims

1. System (100) for magnetic induction treatment comprising:
- at least a magnetic field dispenser (110) comprising a power supply circuit (111) for rechargeable batteries, a receiving and generating unit (112) which receives one or more signals and transmits a current into windings of conductive material (113) to generate one or more Extreme Low Frequency, ELF, magnetic fields with a frequency between 0.1 and 250Hz, and an intensity between 1µT and 220µT; said dispenser (110) having the shape of a parallelepiped and a width of between 20mm and 110mm, a length of between 30mm and 150mm and a thickness of between 10mm and 40mm; said dispenser (110) being inscribed in an elliptical ring containing inside said windings of conductive material (113); said elliptical ring having a major axis between 40mm and 160mm and a minor axis between 30mm and 120mm;
- a web portal and/or application (200) for the purchase and/or acquisition, and the management of treatments (10) including:
∘ a marketplace section (210) in which, by prescription, said treatments (10) are purchased and/or obtained;
∘ a virtual blister section (220) in which the treatments (10) are stored and from which one or more treatments (10) are selected for their administration;
∘ a treatment section (230) in which the treatment (10) being administered is monitored;
∘ a management section (240) in which the start and interruption of a treatment (10) is programmed at specific times;
- at least a control unit (120) comprising an interface (121) for managing magnetotherapy treatments (10), a communication unit (122) configured to send inputs to said receiving and generating unit (112) of the dispenser (110) to start one or more treatments (10); said control unit (120) being configured to communicate with multiple dispensers (110) managing different treatments (10) on different users (1) from said web portal and/or application (200);
- a connection unit (123) for connecting to said web portal and/or application (200);
- user sensors (350) for measuring physiological and biochemical parameters of a user (1); said user sensors (350) configured to send data on the physiological and biochemical parameters monitored, to said web portal and/or application (200) via a transmission unit (115) included in said dispenser (110) which is configured to send the data via Bluetooth, Wi-Fi and/or other wireless communication protocol, wherein the web portal and/or application (200) is programmed to continuously analyze and compare the parameters monitored with said user sensors (350) and automatically interrupt the delivery of the treatment (10) when the parameters are outside established ranges;
- said system (100) being configured to administer magnetic induction therapies to one or more users (1) via one or more treatments (10).

2. System (100) according to claim 1, wherein said dispenser (110) and said control unit (120) are included in a single device (130).

3. System (100) according to any one of the preceding claims, wherein said control unit (120) is a computer, a smartphone or a tablet.

4. System (100) according to any one of the preceding claims, wherein said dispenser (110) is insertable into wearable clothing and accessories.

5. System (100) according to any one of the preceding claims, comprising a wearable modular suit (300) comprising parts (310) that can be coupled together via one or more joining connectors (320), in said parts (310) are placed said windings of conductive material (113) arranged externally to the dispenser (110), and said parts (310) are connected to said dispenser (110) with one or more power connectors (330).

6. System (100) according to the preceding claim 5, wherein the parts (310) of said wearable modular suit (300) are connected to each other and to said dispenser (110) via a connection cable (340).

7. System (100) according to any one of claims 5-6, wherein the dispenser (110) comprises several inputs (114) in which one or more connection cables (340) are connected in order to use different treatments (10) with different parts (310) of said wearable modular suit (300).

8. Use of the system (100) according to any one of claims 1-3, in exposing water, preferably drinking water, to electromagnetic fields.

9. System (100) according to any one of claims 1-7, wherein said treatment (10) is in the form of an NFT created with blockchain technology and/or with connection to a smart contract.

10. The system (100) according to previous claim 2 or previous claim 3 when depending on claim 2, wherein said dispenser (110) or said single device (130) is inserted into a mobile device such as a smartphone.

11. System (100) according to any one of claims 1-7, 9 or 10 wherein said connection unit (123) comprises a housing for a SIM card and a SIM card inserted therein.

12. System (100) according to any one of claims 1-3, wherein said dispenser (110) is implantable inside living organisms in order to administer said treatments (10) remotely.

## Patentansprüche

1. System (100) zur Behandlung mittels magnetischer Induktion, das aufweist:
- zumindest einen Magnetfeldspender (110), der eine Stromversorgungsschaltung (111) für wiederaufladbare Batterien, eine Empfangs- und Erzeugungseinheit (112), die ein oder mehr Signale empfängt und einen Strom in Wicklungen aus leitfähigem Material (113) sendet, um ein oder mehr Magnetfelder im Extrem-Niederfrequenzbereich ("Extreme Low Frequency"; ELF) mit einer Frequenz zwischen 0,1 und 250 Hz und einer Intensität zwischen 1 µT und 220 µT zu erzeugen; wobei der Spender (110) die Form eines Parallelepipeds und eine Breite zwischen 20 mm und 110 mm, eine Länge zwischen 30 mm und 150 mm und eine Dicke zwischen 10 mm und 40 mm aufweist; wobei der Spender (110) in einen elliptischen Ring eingeschrieben ist, der in seinem Inneren die Wicklungen aus leitfähigem Material (113) enthält; wobei der elliptische Ring eine Hauptachse zwischen 40 mm und 160 mm und eine Nebenachse zwischen 30 mm und 120 mm aufweist;
- ein Webportal und/oder eine Anwendung (200) für den Kauf und/oder Erwerb und die Verwaltung von Behandlungen (10), das/die enthält:
∘ einen Marktplatzbereich (210), in dem die Behandlungen (10) durch Rezept gekauft und/oder bezogen werden;
∘ einen Virtueller-Blister-Bereich (220), in dem die Behandlungen (10) gespeichert werden und aus dem eine oder mehr Behandlungen (10) zu ihrer Verabreichung ausgewählt werden;
∘ einen Behandlungsbereich (230), in dem die gerade verabreichte Behandlung (10) überwacht wird;
∘ einen Verwaltungsbereich (240), in dem der Beginn und die Unterbrechung einer Behandlung (10) zu bestimmten Zeiten programmiert werden;
- zumindest eine Steuereinheit (120), die eine Schnittstelle (121) zum Verwalten von Behandlungen (10) mittels Magnetfeldtherapie, eine Kommunikationseinheit (122), die dazu ausgebildet ist, Eingaben an die Empfangs- und Erzeugungseinheit (112) des Spenders (110) zu senden, um eine oder mehr Behandlungen (10) zu starten, aufweist; wobei die Steuereinheit (120) dazu ausgebildet ist, von dem Webportal und/oder der Anwendung (200) aus mit mehreren Spendern (110), die verschiedene Behandlungen (10) an verschiedenen Nutzern (1) verwalten, zu kommunizieren;
- eine Verbindungseinheit (123) zum Verbinden mit dem Webportal und/oder der Anwendung (200);
- Nutzer-Sensoren (350) zum Messen physiologischer und biochemischer Parameter eines Nutzers (1); wobei die Nutzer-Sensoren (350) dazu ausgebildet sind, Daten zu den überwachten physiologischen und biochemischen Parametern über eine in dem Spender (110) enthaltene Übertragungseinheit (115), die dazu ausgebildet ist, die Daten über Bluetooth, Wi-Fi und/oder ein anderes drahtloses Kommunikationsprotokoll zu senden, an das Webportal und/oder die Anwendung (200) zu senden, wobei das Webportal und/oder die Anwendung (200) so programmiert ist, dass es die mit den Nutzer-Sensoren (350) überwachten Parameter kontinuierlich analysiert und vergleicht und die Abgabe der Behandlung (10) automatisch unterbricht, wenn die Parameter außerhalb festgelegter Bereiche liegen;
- wobei das System (100) dazu ausgebildet ist, einem oder mehr Nutzern (1) über eine oder mehr Behandlungen (10) Magnetinduktionstherapien zu verabreichen.

2. System (100) nach Anspruch 1, wobei der Spender (110) und die Steuereinheit (120) in einem einzelnen Gerät (130) enthalten sind.

3. System (100) nach einem der vorstehenden Ansprüche, wobei die Steuereinheit (120) ein Computer, ein Smartphone oder ein Tablet ist.

4. System (100) nach einem der vorstehenden Ansprüche, wobei der Spender (110) in tragbare Kleidung und Accessoires einsetzbar ist.

5. System (100) gemäß einem der vorstehenden Ansprüche mit einem tragbaren modularen Anzug (300), der Teile (310), die über einen oder mehr Verbinder (320) miteinander gekoppelt werden können, aufweist, wobei in den Teilen (310) die Wicklungen aus leitfähigem Material (113), die außerhalb des Spenders (110) angeordnet sind, platziert sind und die Teile (310) mit einem oder mehr Stromanschlüssen (330) mit dem Spender (110) verbunden sind.

6. System (100) gemäß dem vorstehenden Anspruch 5, wobei die Teile (310) des tragbaren modularen Anzugs (300) über ein Verbindungskabel (340) miteinander und mit dem Spender (110) verbunden sind.

7. System (100) nach einem der Ansprüche 5-6, wobei der Spender (110) mehrere Eingänge (114) aufweist, an denen ein oder mehr Verbindungskabel (340) angeschlossen sind, um verschiedene Behandlungen (10) mit verschiedenen Teilen (310) des tragbaren modularen Anzugs (300) anzuwenden.

8. Verwendung des Systems (100) gemäß einem der Ansprüche 1-3, um Wasser, vorzugsweise Trinkwasser, elektromagnetischen Feldern auszusetzen.

9. System (100) gemäß einem der Ansprüche 1-7, wobei die Behandlung (10) in Form eines NFT, das mit Blockchain-Technologie und/oder mit Verbindung zu einem Smart Contract erzeugt wurde, vorliegt.

10. System (100) gemäß dem vorstehenden Anspruch 2 oder dem vorstehenden Anspruch 3, sofern abhängig von Anspruch 2, wobei der Spender (110) oder das einzelne Gerät (130) in ein mobiles Gerät wie etwa ein Smartphone eingesetzt ist.

11. System (100) gemäß einem der Ansprüche 1-7, 9 oder 10, wobei die Verbindungseinheit (123) ein Gehäuse für eine SIM-Karte und eine darin eingesetzte SIM-Karte aufweist.

12. System (100) gemäß einem der Ansprüche 1-3, wobei der Spender (110) in lebende Organismen implantierbar ist, um die Behandlungen (10) ferngesteuert zu verabreichen.

## Revendications

1. Système (100) pour traitement par induction magnétique comprenant :
- au moins un distributeur de champ magnétique (110) comprenant un circuit d'alimentation électrique (111) pour batteries rechargeables, une unité de réception et de génération (112) qui reçoit un ou plusieurs signaux et transmet un courant dans des enroulements en matériau conducteur (113) pour générer un ou plusieurs champs magnétiques à extrême basse fréquence, ELF, avec une fréquence comprise entre 0,1 et 250 Hz, et une intensité comprise entre 1 µT et 220 µT ; ledit distributeur (110) ayant la forme d'un parallélépipède et une largeur comprise entre 20 mm et 110 mm, une longueur comprise entre 30 mm et 150 mm et une épaisseur comprise entre 10 mm et 40 mm ; ledit distributeur (110) étant inscrit dans un anneau elliptique contenant à l'intérieur lesdits enroulements en matériau conducteur (113) ; ledit anneau elliptique ayant un grand axe compris entre 40 mm et 160 mm et un petit axe compris entre 30 mm et 120 mm ;
- un portail web et/ou une application (200) pour l'achat et/ou l'acquisition, et la gestion de traitements (10) incluant :
∘ une section de marché (210) dans laquelle, sur ordonnance, lesdits traitements (10) sont achetés et/ou obtenus ;
∘ une section d'emballage-coque virtuelle (220) dans laquelle les traitements (10) sont stockés et à partir de laquelle un ou plusieurs traitements (10) sont sélectionnés pour leur administration ;
∘ une section de traitement (230) dans laquelle le traitement (10) administré est surveillé ;
∘ une section de gestion (240) dans laquelle le démarrage et l'interruption d'un traitement (10) sont programmés à des moments spécifiques ;
- au moins une unité de commande (120) comprenant une interface (121) pour la gestion de traitements de magnétothérapie (10), une unité de communication (122) configurée pour envoyer des entrées à ladite unité de réception et de génération (112) du distributeur (110) afin de démarrer un ou plusieurs traitements (10) ; ladite unité de commande (120) étant configurée pour communiquer avec plusieurs distributeurs (110) gérant différents traitements (10) sur différents utilisateurs (1) depuis ledit portail web et/ou ladite application (200) ;
- une unité de connexion (123) pour se connecter audit portail web et/ou à ladite application (200) ;
- des capteurs utilisateur (350) permettant de mesurer des paramètres physiologiques et biochimiques d'un utilisateur (1) ; lesdits capteurs utilisateur (350) étant configurés pour envoyer des données sur les paramètres physiologiques et biochimiques surveillés, audit portail web et/ou à ladite application (200) via une unité de transmission (115) incluse dans ledit distributeur (110) qui est configurée pour envoyer les données via Bluetooth, Wi-Fi et/ou autre protocole de communication sans fil, dans lequel le portail web et/ou l'application (200) est programmé(e) pour analyser et comparer en continu les paramètres surveillés avec lesdits capteurs utilisateur (350) et interrompre automatiquement la délivrance du traitement (10) lorsque les paramètres sont en dehors de plages établies ;
- ledit système (100) étant configuré pour administrer des thérapies par induction magnétique à un ou plusieurs utilisateurs (1) via un ou plusieurs traitements (10).

2. Système (100) selon la revendication 1, dans lequel ledit distributeur (110) et ladite unité de commande (120) sont inclus dans un seul dispositif (130).

3. Système (100) selon l'une des revendications précédentes, dans lequel ladite unité de commande (120) est un ordinateur, un smartphone ou une tablette.

4. Système (100) selon l'une des revendications précédentes, dans lequel ledit distributeur (110) est insérable dans des vêtements et accessoires portables.

5. Système (100) selon l'une des revendications précédentes, comprenant une combinaison modulaire portable (300) comprenant des parties (310) pouvant être couplées ensemble via un ou plusieurs connecteurs de jonction (320), dans lesdites parties (310) sont placés lesdits enroulements en matériau conducteur (113) agencés de façon externe au distributeur (110), et lesdites parties (310) sont connectées audit distributeur (110) par un ou plusieurs connecteurs d'alimentation (330).

6. Système (100) selon la revendication 5 précédente, dans lequel les parties (310) de ladite combinaison modulaire portable (300) sont connectées les unes aux autres et audit distributeur (110) via un câble de connexion (340).

7. Système (100) selon l'une des revendications 5-6, dans lequel le distributeur (110) comprend plusieurs entrées (114) dans lesquelles un ou plusieurs câbles de connexion (340) sont connectés afin d'utiliser différents traitements (10) avec différentes parties (310) de ladite combinaison modulaire portable (300).

8. Utilisation du système (100) selon l'une des revendications 1 à 3, pour exposer de l'eau, de préférence de l'eau potable, à des champs électromagnétiques.

9. Système (100) selon l'une des revendications 1 à 7, dans lequel ledit traitement (10) est sous la forme d'un jeton non fongible (NFT) créé avec la technologie de chaîne de blocs et/ou en connexion avec un contrat intelligent.

10. Système (100) selon la revendication 2 précédente ou la revendication 3 précédente lorsqu'elle dépend de la revendication 2, dans lequel ledit distributeur (110) ou ledit seul dispositif (130) est inséré dans un dispositif mobile tel qu'un smartphone.

11. Système (100) selon l'une des revendications 1 à 7, 9 ou 10, dans lequel ladite unité de connexion (123) comprend un logement pour une carte SIM et une carte SIM insérée dans celui-ci.

12. Système (100) selon l'une des revendications 1 à 3, dans lequel ledit distributeur (110) est implantable à l'intérieur d'organismes vivants afin d'administrer à distance lesdits traitements (10).
